# EUROPEAN PATENT SPECIFICATION

(11) **EP 1 492 446 B1**
(45) Date of publication and mention of the grant of the patent: **09.05.2012**
(21) Application number: 03719025.3
(22) Date of filing: 02.04.2003
(51) Int. Cl.: A61B 5/151, G01N 33/487, A61B 5/145

(54) **INTEGRATED SAMPLE TESTING METER**
INTEGRIERTES TESTGERÄT FÜR PROBEN
APPAREIL INTEGRE D'ANALYSE D'ECHANTILLONS

(30) Priority: 02.04.2002 GB 0207610
(43) Date of publication of application: 05.01.2005
(73) Proprietor: Lifescan Scotland Ltd, Inverness, IV2 3ED, Scotland (GB)
(72) Inventor: GRIFFITH, Alun, Wyn, Inverness, IV2 3XQ (GB); MAY, Keith, Ashford, Kent TN26 3PU (GB); DISTON, Andrew, Stephen, Cambridge CB4 6AJ (GB); DROUGHT, Nicholas, Andrew, Cambridge CB4 6DS (GB); WILSON, Donna, Joy, New York, NY 10128 (US)
(74) Representative: Tunstall, Christopher Stephen
(86) International application number: PCT/IB2003/001844
(87) International publication number: WO 2003/082091

(56) References cited:
- EP-A- 1 112 717
- EP-A2- 0 732 590
- WO-A-01/17425
- WO-A-99/59657
- DE-A- 4 234 553
- DE-A- 19 819 407
- US-A- 6 071 294
- US-A1- 2002 002 326
- US-B1- 6 315 738

## Description

### Field of the Invention

The present invention relates to an integrated sample testing meter for use in sampling and analyzing analytes, particularly glucose, in fluids such as blood or interstitial fluid.

### Background of the Invention

Glucose monitoring is a fact of everyday life for diabetic individuals. The accuracy of such monitoring can literally mean the difference between life and death. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. A number of glucose meters are currently available which permit an individual to test the glucose level in a small sample of blood.

Many of the glucose meter designs currently available make use of a disposable test strip which, in combination with the meter, electrochemically or photometrically measures the amount of glucose in the blood sample. To use these meters, the user first punctures a finger or other body part using a puncture means, such as a lancet, to produce a small sample of blood or interstitial fluid. The sample is then transferred to a disposable test strip. The inconvenience of taking several measurements a day, as well as the pain inflicted by currently available puncture means, often discourage disciplined and frequent testing.

While the fingertip is generally used for sampling blood, due to the rich capillary bed of the skin of the fingertip, the fingertip is also particularly sensitive to pain, due to a rich supply of pain receptors in the finger tip as well. When a puncture is too deep, too close to a recent puncture or not deep enough and requires an additional puncture, the pain increases significantly. Pain may also be increased if the puncture means penetrates slowly or is withdrawn slowly. Furthermore, the user may be forced to make a larger puncture than is necessary to form a sufficient amount of blood, due to losses during the transfer between the puncture site and the test strip.

The process of measuring blood glucose levels requires several steps and several different accessories, including a puncturing device, a puncture means, a supply of test strips and a glucose meter. Each accessory has a different function. The user must have a flat surface available to unpack and lay down the accessories within easy reach. This, by itself, poses a challenge for those who need to take measurements while participating in outdoor activities. Flat surfaces are often not available and this can discourage a person from taking a measurement. This can be disadvantageous because blood glucose levels may change significantly during an outdoor activity.

Even if a user can find a flat surface, the user has to carry out the following steps. The user: charges the puncturing device with a fresh puncture means; opens a vial of strips; removes a strip; inserts the strip into the meter; re-closes the vial; checks for the correct calibration code on the meter; picks up the puncturing device; lances the skin of the finger or other body part; lays down the puncturing device; squeezes or massages the finger to yield an adequate blood sample; transfers the sample to the test strip for analysis; waits for the meter to analyze the sample; removes the strip from the test meter; discards the strip; and finally re-packs all of the accessories. As set forth above, the standard procedure for taking a glucose measurement requires the use of multiple, separate components and the execution of a number of steps requiring manual user intervention.

DE 19819407 discusses a measuring device in which test strips are stored in a part of a container, are brought via a mechanism into the corresponding measuring position and, once the measurement has been taken, are transferred to a collection compartment.

Generally, the user is required to transfer a small volume of sample to a sample-receiving area on the test strip. Generally, test strips are quite small and the sample-receiving area is therefore even smaller. This transfer step is a difficult task for many users. Moreover, there has recently been a trend towards the use of test strips requiring ever smaller amounts of sample. (This allows the use of smaller punctures and therefore less painful puncturing.) However, the use of smaller samples increases the difficulty in transferring the sample to the sample-receiving area on the test strip. This is especially difficult for users with poor eyesight, a common complication for diabetics.

The pain, inconvenience, cost, slowness, complexity and discreteness of taking a blood glucose measurement are barriers to the frequent monitoring of glucose levels. Patients often do not comply with doctor recommendations to frequently test glucose levels due to the numerous obstacles involved.

It is an aim of the present invention to provide, at least in part, a solution to the above problems.

### Summary of the Invention

The present invention provides an integrated sample-testing meter according to claim 1.

In use, after the puncture has been made, the user moves the meter to a second position where the sample-receiving area of the test strip is located in the drop and receives a sample from the fluid drop. The sensor then analyzes the sample.

Preferably, the meter of the present invention will include electrical or electronic circuitry for controlling its operation. Such circuitry may be hard-wired or may comprise a microcomputer or similar device. Such circuitry will in particular include all the components of the sensor and will be arranged to carry out the analysis of the sample.

Preferably, the circuitry also includes a visual display unit from which the user can read out the result of any particular test. The display may also be adapted to provide a display of the data, as explained in more detail below.

Preferably, the circuitry includes means, such as a touch sensitive display, control buttons or a microphone and voice activated software, for entering data into the meter.

Preferably, the meter includes a pressure device arranged to facilitate the formation of a drop of fluid around the puncture.

The pressure device may comprise a pump adapted to apply a negative pressure to a volume in the meter having an aperture for location on the skin of the user. Advantageously, the part of the meter forming the aperture through which the puncture means extends is made of a non-slip material so that the meter can be more securely located on the user's skin during the puncturing operation.

Preferably, however, the pressure device comprises a pressure ring arranged to be located, in use, on the user's skin and to apply pressure at the edges of the ring to increase the amount of fluid available at the centre of the ring. Advantageously, the pressure ring is made of a non-slip material so that the meter can be more securely located on the user's skin during the puncturing operation.

The pressure ring may be shaped to conform to the shape of the area of the skin to which it is to be applied. For instance, if the meter is intended for use on the forearm, the pressure ring will be generally planar. However, if the meter is intended for use on a finger, the pressure ring will be curved.

Preferably, the pressure ring has a multi-contoured surface to increase the pressure gradient from the outside to the inside of the ring.

Advantageously, the pressure ring is part of a cap which covers the puncture means in its retracted position. Preferably, the cap includes a means, such as a sidewall, which co-operates with the drive train to ensure that the puncture means travels along approximately the same path on each activation of the drive train.

The cap may be an integral part of the housing. Preferably, however, the cap is detachably mounted on the housing. This may be achieved by use of screw-thread or bayonet type fixings, by use of a snap fit connection or by a hinged connection.

If desired, the meter can include at least two interchangeable caps, for instance a cap for forearm use and a cap for finger use.

The puncture means may be any of the types of puncture means at present in use in the art. The term "puncture means" includes lancets and finger-sticking devices of the type known in the art. Preferably, the puncture means is removably attached to the drive train so that the puncture means can be disposed of after one or several uses.

Preferably, the drive train is spring driven. Alternatively, the drive train is driven electromagnetically. The drive train is arranged such that, on actuation, the puncture means moves to the extended position and is retracted.

Preferably, the drive train includes an adjustment screw which allows the user to set the extended position of the puncture means. This enables the user to calibrate the operation of the meter such that his or her skin is punctured sufficiently to allow a large enough drop of fluid to form without causing too much pain.

Advantageously, the operation of the adjustment screw is arranged such that the distance of travel of the puncture means remains constant, however much the extended position of the puncture means is changed. This ensures that the amount of pain experienced by the user does not increase disproportionately to the depth of puncture.

Where the meter includes a cap, it is preferable, as noted above, that the cap provides a means for guiding the drive train so that the puncture means punctures the skin at approximately the same place on each actuation of the drive train.

Preferably, the test strip cartridge comprises a cartridge housing defining a cavity configured to receive a stack of test strips, a partially detachable cartridge cap and a means for moving the stack of test strips towards the cartridge cap.

The test strips used for some measurements are air- or moisture- sensitive. It is therefore preferred that the cartridge includes a seal for sealing the cartridge cap to the cartridge housing when the cartridge cap is fully engaged with the cartridge housing. The seal may be on the cartridge cap or on the cartridge housing.

In use; upon activation of the meter, the cartridge cap is partially detached from the cartridge housing to allow the first test strip in the stack to be moved by the test strip dispensing system to the sample-receiving position. Once the measurement has been taken, the cartridge cap is preferably manually replaced on the cartridge housing to close the cartridge and seal its contents from atmospheric effects.

Preferably, the cartridge has on it data relating to the calibration code for the strips in the cartridge. The data may be present as visually readable indicia. In this case, the meter must include means, as mentioned above, to allow the user to enter the calibration code into the meter.

Preferably, however, the data on the cartridge are present in machine-readable format, for instance as a bar code or a resistance bridge circuit or stored in an electronic memory module.

If the data are present as a bar code, the meter will include a bar code reader. This may be a scanning reader or a stationary reader. A scanning reader will be more complicated but can be used when the cartridge is fitted in the meter. A stationary reader is less complicated but can only be used as the cartridge is inserted into or taken out of the meter.

If the data are present in an electronic memory module, this may comprise a read-only memory (ROM), or a rewritable memory, such as an EPROM or EEPROM.

Preferably, the data also include a unique number identifying the specific cartridge, the number of strips originally present in the cartridge, the expiry date for the cartridge, different calibration factors for different sources of fluid (neonatal, arterial or venous blood, for instance), an acceptable performance range and any other relevant information, preferably in machine-readable format, to assist in operation of the meter.

Where the memory module on the cartridge is rewritable, the meter may be arranged to write back into the memory module information such as the number of strips used, the date the cartridge was first used, the length of time the cartridge has remained open and the date, time and result of each test that was carried out with a strip from the cartridge.

Preferably, the test strip dispensing system includes a slider adapted to engage with only one of the test strips in the cartridge and move it to the sample-receiving position.

Advantageously, the meter includes a feeding channel which receives the strip from the cartridge and guides it to the sample-receiving position.

Preferably, the feeding channel includes a step arranged such that, when the strip has been moved past the step, the strip drops, or is forced, into the step, thereby preventing the strip from moving back towards the cartridge.

Preferably, the strip is forced into the step by springs located on the meter. Advantageously, the springs are also electrically conductive and are arranged to make electrical contact with electrodes or a conductive bar on the strip (see below).

Alternatively, the strips may be provided with cut-outs, for instance of triangular shape, which mate with spring-biased abutments which fit into the cut-outs to hold the strip in its sample-receiving position.

Advantageously, the meter includes an ejection means for ejecting a used test strip from the meter once a test has been completed. Preferably, where the cartridge includes a cartridge cap, the ejection means is operated as the cartridge cap is closed.

Preferably, the meter includes a deviator which prevents the test strip dispensing system moving a further test strip into the sample-receiving position while a first test strip is still in position. This is an advantageous feature as it allows the user to carry out a number of puncturing operations with the same strip in position, since, in some cases, it takes a number of puncturing operations, if necessary with adjustment of the penetration depth, to produce a sufficiently large drop of fluid.

Preferably, the deviator operates in conjunction with the cartridge cap. While the cartridge cap is partially detached from the cartridge housing, the deviator blocks the normal path for the test strip dispensing system, such as the slider, and causes it to enter the cartridge cap rather than the regular path into the cartridge.

Preferably, the meter includes a means for verifying that a strip is in the sample-receiving position. This may comprise a reflectance meter. Generally, test strips are more or less reflective than the surfaces of the feeding channel. Therefore, a change in reflectance will indicate that a test strip is in position.

Preferably, however, the verifying means comprises an electrical system. At its simplest, each strip may have on it a conductive bar arranged to short out two electrodes on the meter. This arrangement is useful for strips arranged to carry out photometric measurements.

Strips which are arranged to carry out electrochemical measurements already include electrode systems. Thus, the verifying means may include electrical contacts on the meter which contact the electrodes on the strip. Advantageously, as noted above, the electrical contacts on the meter are preferably spring-loaded and are positioned to force the strip into the step in the feeding channel.

Advantageously, the verifying means is also used to activate fully the circuitry in the meter. The meter may normally be in a low power mode, where the only active circuitry is that used to control the verifying means. Once the verifying means has indicated that a strip is present, the meter can then automatically switch to high power mode where all its relevant circuits are functioning.

Preferably, the verifying means is also arranged to start a timer in the circuitry of the meter. The timer is stopped by the ejection of a used strip from the meter, preferably by closure of the cartridge cap. This allows the circuitry to determine the length of time the cartridge has been open to the atmosphere. Advantageously the circuitry is arranged to sum the total time that the cartridge has been open and to produce a warning signal, such as an audible tone or a visible signal, if the total exceeds a pre-set maximum.

Preferably, the circuitry in the meter also counts the number of strips dispensed from each cartridge. Advantageously, the circuitry is designed to provide a warning signal, such as an audible tone or a visible signal, when the number of strips remaining in the cartridge is low.

Where the cartridge has on it data relating to an acceptable performance range, preferably the circuitry is arranged so that, if a control test gives a result outside the performance range, the meter is disabled while that cartridge is in the meter. This arrangement ensures that, if the strips in a particular cartridge have deteriorated, they cannot be used.

As noted above, the cartridge preferably includes a rewritable memory module and the circuitry in the meter is arranged to write back to the cartridge memory module useful information, such as the number of strips remaining in the cartridge and the length of time the cartridge has been open to the atmosphere. The rewriting function is particularly useful where a user is likely to be away from his normal environment for a length of time which would require the use of more strips than are present in a cartridge. In such cases, a user is likely to remove the old cartridge and insert a new, full cartridge. Once the new cartridge has been used up, the user may insert the old cartridge, even if it is out of date. As long as the meter can read the data on the old cartridge, it will be able to decide whether use of the old cartridge should be allowed.

Moreover, the provision of a rewritable memory module enables other possible uses. For instance, data on time and date of use and result of measurement may be written into the cartridge's memory module. The used cartridge could then be returned to the user's health care provider who could then study the data to determine whether the user is complying with his treatment and monitoring regime. Alternatively, the used cartridges could be returned to the manufacturer to enable a general study of use to be carried out. These data, illustrating the effective use of strips, may provide a tool for health care insurers to verify the actual usage of the strips they reimbursed.

Preferably, the meter is activated manually by use of a single movement, for instance of a multi-functional assembly carried by the housing. The assembly may include a lever pivoted to the housing. Preferably, however, the assembly includes a slidable knob which slides to cock the drive train and move a strip into the sample-receiving position.

The movement of the assembly may also activate all the meter's circuitry.

The drive train may be fired either by further movement of the lever or, preferably, by actuation of a trigger.

It can thus be seen that the use of the integrated sample testing meter of the present invention can be very simple. If desired, the user can replace an existing puncture means with a new one. The meter can then be cocked by use of the assembly. This also moves a strip into the sample-receiving position. Movement of the lever or receipt of a strip in the sample receiving position also activates all the meter's circuitry. Then, the user places the appropriate part of the meter, such as the aperture or the cap, on his or her skin and activates the trigger.

If the first activation of the trigger does not cause the production of a sufficiently large drop of fluid, the meter can be cocked, positioned and fired again, if necessary a number of times, without the need to insert a new strip.

Once sufficient fluid has accumulated around the puncture, the user moves the meter to the second position in which the strip is placed in the drop and its sample-receiving area takes up a sample of the fluid.

Thus, the use of the meter of the present invention avoids most of the steps presently required and in particular avoids those steps where manual dexterity and good eyesight are advantageous.

Preferably, the meter is adapted to produce and analyze a sample of blood or interstitial fluid, in particular to analyze a blood sample for glucose levels. Strips adapted to carry out such measurements are well known in the art. These may be electrochemical or photometric strips.

Advantageously, the strips are adapted to carry out electrochemical analyses and the circuitry in the meter is arranged to contact the electrodes in such strips.

Therefore, in a preferred embodiment the present invention provides an integrated blood glucose testing meter. The integrated meter of this aspect of the present invention allows for a simple, one-step glucose monitoring process, and significantly reduces the obstacles involved in frequent glucose monitoring. The integrated meter provides for the automated dispensing and positioning of a test strip and puncturing of a user in a repeatable manner. After user-initiated transfer of a blood sample to the test strip, automated analysis of the blood sample takes place.

There is provided (but not claimed) an integrated method of sampling and testing a blood glucose level or other analyte in a bodily fluid. The integrated method comprises loading a test strip cartridge into an integrated testing meter, activating an assembly on the testing meter to cock a puncture means drive train and move a test strip into a sample-receiving position, pressing the integrated testing meter on the skin of a user and pressing a trigger of the testing meter to drive a puncture means into the skin in order to form a drop of blood or other fluid on the skin surface. The user moves the meter such that the test strip can absorb a required amount of blood or other fluid for an automated analysis of the sample by the integrated testing meter.

### Brief Description of the Drawings

These and other features and advantages of the present invention will be more fully understood by reference to the following detailed description in conjunction with the attached drawings in which like reference numerals refer to like elements through the different views.
Figure 1 is a perspective view of an integrated blood and testing meter according to the present invention with a strip in the sample-receiving position.
Figure 2 is a perspective view of the meter of Figure 1 with the lancet cover in its open position.
Figure 3 is a perspective view of the meter of Figure 1 with the subhousing in its open position.
Figure 4 is a schematic view of part of the interior of a feeding channel with a strip in place.
Figure 5 illustrates a test strip design suitable for use in the present invention.
Figure 6 is a schematic representation of the electronics which can be incorporated in an integrated meter in accordance with the present invention.

### Detailed Description of the Invention

The present invention provides an integrated meter for sampling and analyzing a sample of bodily fluid, such as blood, including a disposable test strip cartridge having a stack of test strips disposed therein. The present invention facilitates the monitoring of, for instance, blood glucose levels by integrating into a single meter the steps involved in sampling and analyzing blood into a simple process employing a single meter.

The present invention will be described below relative to an illustrative embodiment. Those skilled in the art will appreciate that the present invention may be implemented in a number of different applications and embodiments and is not specifically limited in its application to the particular embodiment depicted herein.

The present invention will be discussed below in connection with sampling blood, although those of ordinary skill will recognize that other types of fluid can also be used.

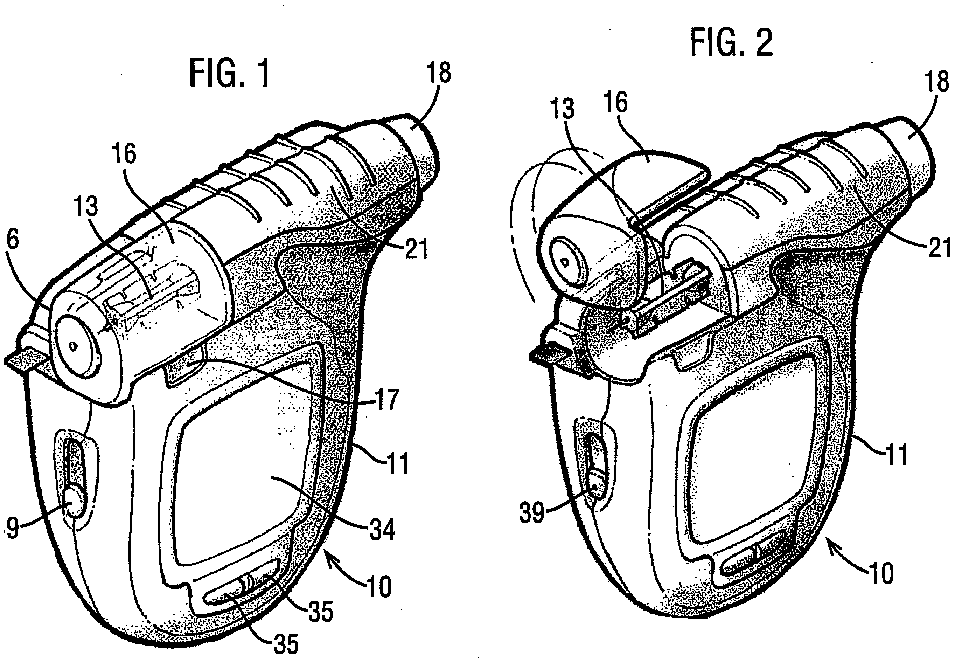

Figures 1 to 3 illustrate an integrated blood glucose sampling and testing meter 10 according to an illustrative embodiment of the present invention. This meter is designed to carry out electrochemical analysis of a blood sample. However, if desired, the same mechanical parts could be used in connection with photometric analyses. The sampling and testing meter comprises a modular housing 11 encompassing an integrated system for expressing and subsequently analyzing a sample. The meter 10 includes an assembly for puncturing the skin of a user to express a drop of blood on the surface of the skin. The assembly includes a lancet 13 as the puncture means and a drive train 14 for driving the lancet into and out of the skin.

A transparent cap 16 is attached to the housing 11 by a hinge at the proximal end of the device 10. The housing 11 includes a recess 17 for enabling the cap 16 to be moved to the open position shown in Figure 2. In this position, the lancet can be removed and replaced. The cap 16 also includes an aperture to allow passage of the lancet 13 through the cap 16 and into the skin of the user. The cap 16 can have a multi-contoured surface in order to promote, enhance or facilitate the expression of blood by pressing the device onto the skin. The assembly further includes a depth adjustment knob 18 situated at the distal end of the drive train opposite the lancet. Rotation of the depth adjustment knob decreases or increases the puncture depth of the lancet. The depth adjustment knob regulates or adjusts the puncture depth in accordance with known techniques.

A test strip cartridge 19 is loaded into the meter 10 and includes a stacked supply of test strips disposed within a cavity or hollow interior of the cartridge housing. The test strip cartridge is adapted to dispense individual test strips to a feeding channel 20. The outlet of the feeding channel leads to the exterior of the meter 10.

The housing may include an internal wall that defines the inner side of the cap 16. Alternatively, the housing wall can have a frusto-conical or funnel shape, or any other suitable shape, for precisely controlling the movement of the lancet.

The cap is precisely dimensioned such that the lancet 13 slidably passes through the cap. In this arrangement, the lancet 13 is precisely positioned at about the same location each time it is deployed. Correspondingly, each test strip is precisely positioned at about the same location each time one is moved from the cartridge to the sample-receiving position.

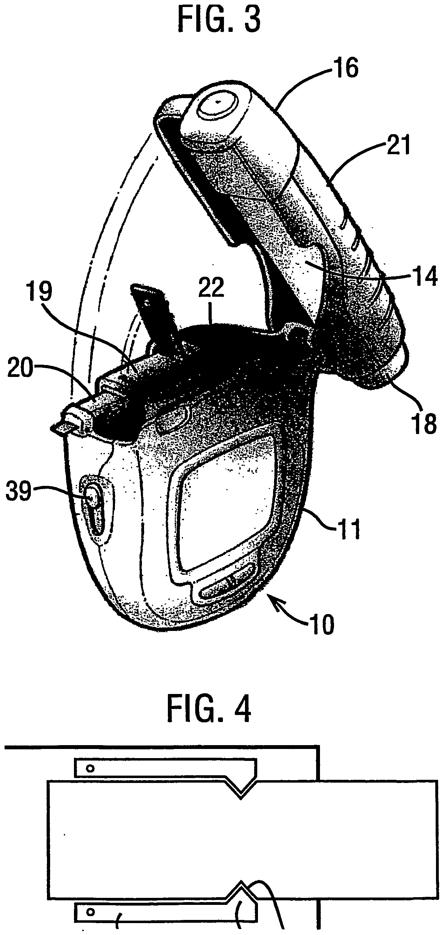

The test strip cartridge 19 comprises a replaceable and disposable portion of the sampling and testing meter. When the supply of test strips is depleted or expired, the user may open the meter 10, as shown in Figure 3, remove the used test strip cartridge 19 and insert a new test strip cartridge containing a fresh supply of test strips. The details of the test strip cartridge 19 are described in depth below.

A strip dispensing system operates in co-operation with the test strip cartridge 19 to dispense test strips one-by-one through the feeding channel 20 and into the sample-receiving position to effect the sampling and analysis of a blood sample. According to the illustrated embodiment, when a user slides knob 21 of the meter 10 away from the cap 16, the strip dispensing system pushes the foremost test strip in the stack out of the test strip cartridge and into the feeding channel. According to a preferred embodiment, knob 21 performs an additional function of simultaneously cocking the lancet assembly to prepare the lancet assembly for puncturing the skin of a user when the user presses the cap 16 to the skin. The workings of the strip dispensing system and knob 21 are described in further detail below.

To enable electrochemical analysis of the sample, the meter further includes electrical contacts situated in the feeding channel 20 and configured to contact electrodes formed on the test strip. The electrical contacts connect to electronics 22 located within the modular housing 11 of the sampling and testing meter. The electronics are arranged such that, once the contacts contact the electrodes in the strip, the meter switches from "low" power mode to "high" power mode.

The test strip generates electrochemical signals that are passed by the electrical contacts to the housing electronics. The electronics process the signal and calculate the glucose level or other electrochemically detectable analyte of the blood or interstitial fluid that is sampled by the testing device. The electronics transmit instructions for an appropriate display or output regarding the analysis.

As shown in Figure 4, the feeding channel 20 has in it a pair of arms which are biased to move towards each other. Each arm has at its free end a triangular abutment 31. The strips have in them triangular cut-outs 33. When a strip is fed into the feeding channel, the abutments 31 on the arms 30 snap into the cut-outs 33 to hold the strip in the sample receiving position.

Alternatively, the feeding channel may include a step located adjacent the electrical contacts. The electrical contacts are spring biased so that, once a test strip is in the sample-receiving position, the electrical contacts bear on the test strip and locate it securely in the step.

In either manner, the strip is prevented from moving backward away from the sample-receiving position.

The integrated sampling and testing meter 10 includes a visual LCD display 34 for displaying information related to the analysis of the sample. According to the illustrative embodiment, the information in the display includes a measured blood glucose level in a blood sample, as well as the time and date of the measurement. The display may also provide information regarding the number of test strips remaining in the test strip cartridge, the operating temperature, the expiration date of the test strip cartridge, instructions to the user and the like. According to one practice of the invention, test results are stored in memory in the meter and the display 34 allows a user to view prior test results.

The meter also has on its outside buttons 35 which can be used by the user to enter data into the meter's electronics. This may be achieved by using the buttons to navigate through one or more menus displayed on the display 34.

To measure blood glucose levels with the integrated meter 10, a user first slides the knob 21 away from the cap 16 to simultaneously cock the lancet assembly and automatically open the test strip cartridge and to advance a test strip from the cartridge through the feeding channel 20 to the sample-receiving position shown in Figure 1. The user then presses the cap 16 against a body part, such as a finger or forearm. This releases the lancet assembly, which fires the lancet 13 into the skin a predetermined depth and at a precise location. The lancet assembly immediately retracts the lancet from the skin.

The cap 16 includes a pressure ring (not shown) so that, as the meter is pressed onto the skin before, during or after the puncturing has taken place, a drop of blood of the required size forms on the user's skin. The user then moves the meter to bring the sample-receiving area of the strip in the sample-receiving position into contact with the drop. As the blood contacts the sample-receiving area of the test strip, capillary force absorbs blood into the strip for analysis. The user holds the meter firmly against the skin until a sufficient amount of blood is absorbed into the test strip, generally for about 3 to 10 seconds. According to one practice, the meter 10 produces an audible or visible signal to the user indicating that a sufficient blood sample has been collected and that analysis has begun. The user then removes the meter from the skin and the electrochemical analysis of the sample continues until the result is displayed.

The disposable strip cartridge 19 includes a number of components designed to facilitate automatic, one-by-one dispensing of the test strips. The test strip cartridge includes a vial housing, a cartridge housing including a stack of test strips, a cartridge cap and a push-up or biasing mechanism. The stack of test strips comprises about fifty test strips in vertical alignment. However, the test strip cartridge of the present invention is not limited to a stack of fifty test strips and may include any number of stacked test strips.

The push-up mechanism biases the test strip stack towards the cartridge cap such that, when a foremost test strip is removed from the stack, the remaining test strips in the stack advance by one. After the foremost strip is removed from the stack, the next strip in the stack moves up and is ready to be dispensed for a subsequent analysis. The push-up mechanism includes a loader pressing against the last strip in the stack and a biasing element, such as a tensator. The tensator comprises a constant force clock spring that applies a constant pressure to the stack.

The push-up mechanism further includes a tensator retainer to secure a portion of the tensator to the cassette housing. The vial housing further includes notches to releasably lock the cartridge in the modular housing of the meter. When loading the cartridge 19 into the meter, the vial housing clicks unambiguously in place to ensure a precise fit.

The cartridge cap includes a hermetic sealing element to protect the test strips from humidity, which can damage the test strips and compromise test results. Alternatively, the seal can be included in the vial housing where it meets the cap.

According to one practice, the cartridge material itself can have desiccant properties, or desiccants can be disposed in the interior space of the vial. Any humidity that may migrate into the test strip vial is by these materials absorbed and neutralized.

As can be seen from Figure 3, preferably the cap, the drive train, the sliding knob and a strip ejection lever (see below) are in a unitary subhousing which is pivotally attached to the remainder of the housing to allow for the insertion of a cartridge and the removal of used cartridges. The subhousing may be released by operation of release knob 39.

Preferably, the cartridge includes on it a re-writable memory module such as an EPROM or EEPROM chip. In this case, the electronics in the meter will include means for interfacing with the memory module so that the meter can read from and write to the memory module.

The memory module will contain a calibration code for the cartridge and will preferably contain a unique code for the cartridge and its expiry date. It may also contain compensation factors for analyses of different fluids (such as venous, arterial or neonatal blood or interstatial fluid), the number of strips in the cartridge and other relevant information. The electronics in the meter will be set up to use any data stored in the memory module, in particular the calibration code.

The electronics will also be set up to write to the memory module such information as the number of strips used, the length of time the cartridge cap has remained open, the date of first opening the cap, the date and times of each test carried out and the result of each test.

The cartridge may alternatively include such data in other formats, such as in visible characters, as a bar code or as a resistor bridge circuit.

The cartridge cap is releasably locked into place on the cartridge by a cap retainer. To allow for the strip dispensing mechanism in the meter to forward individual strips to the feeding channel 20, the cartridge cap includes a "pop-up" feature. The cartridge cap is flexibly attached to the vial housing by means of side supports, hinges, springs or another suitable mechanism Pushing the cap retainer releases the lock on the cartridge cap and allows the cap to pop up a predetermined amount, thereby allowing the foremost test strip in the stack to be fed to the sample-receiving position.

The strip dispensing system cooperates with the pop-up cartridge cap described above to push the foremost test strip of a test strip stack into the feeding channel 20 in order to position the test strip in the sample-receiving position. As discussed, the strip dispensing system comprises the sliding knob 21.

A more detailed description of the cartridge and a feeder mechanism for use therewith is found in our copending International Patent Application No. PCT/GB02/0207609.9 filed concurrently herewith (Attorney's ref: P030115WO), which claims priority from USSN 60/280321 filed on 29th March, 2001.

Those skilled in the art will recognize that any suitable mechanism may be utilized for forwarding a test strip into a feeding channel and ensuring that the test strip is entirely dispensed. Once in the feeding channel, the test strip is positioned to receive a blood sample for analysis. After the analysis is complete, the user replaces the cartridge cap to re-seal the cartridge, for instance by operation of the ejection lever 36.

The slidable knob 21 further includes means for arming the drive train of the meter 10. According to one practice, the knob further operates to switch on the electronics of the meter 10 to prepare the meter for analysis of a prospective blood sample. According to an alternate embodiment, the electronics include a strip detector for detecting the presence of a test strip in the feeding channel. Thus, when the strip detector detects a strip in proximity to the puncturing site, the electronics switch on.

According to one aspect, the strip dispensing system is designed to ensure that only one test strip is loaded at a time. The strip dispensing system includes a deviator in co-operation with the slider. The strip dispensing system allows only one test strip to be forwarded at a time. After the slidable knob is released and the slider is brought back into its initial position, the deviator automatically rotates into a position to deflect subsequent attempts to load an additional test strip into the feeding channel. The release of the cartridge cap caused by operation of the knob allows the deviator to rotate once the slider is moved back to its idle position. After forwarding one test strip, the slider route is deviated inside the cartridge cap, rather than through the test strip cartridge and into the feeding channel of the meter 10.

When the user presses the cartridge cap closed, the deviator rotates back and resets the strip dispensing system to dispense a new strip. If a test strip is already loaded into the feeding channel 20, additional operation of the slidable knob 21 only serves to cock the lancet assembly and does not load another test strip into the channel. In this manner, the strip dispensing system allows several cocking and puncturing attempts using the same test strip. This feature is particularly useful if the lancet is accidentally discharged or if the puncturing action does not generate a sufficient amount of blood. In this case, the lancet assembly can be re-cocked without wasting a test strip.

The test strip cartridge 19 and the strip dispensing system co-operate with the lancet assembly illustrated in Figure 1 to efficiently and less painfully obtain and analyze a blood sample from a user. As discussed above, operation of the slidable knob 21 simultaneously cocks the lancet assembly and forwards a test strip from the cassette into the feeding channel 20. The drive train for the lancet assembly may comprise a drive tube, a lancet holder slidably mounted in the drive tube for holding the lancet 13, a first spring for urging the lancet holder forward, a second spring for retracting the lancet 13 after the lancet punctures the skin and a depth adjuster knob 17. The lancet assembly further includes the cap 16 having an aperture for guiding the lancet 13 through the aperture to the skin of a user and for shielding the lancet when not in use.

When the slidable knob 21 is operated, the drive tube retracts to arm the lancet assembly, while simultaneously the test strip is fed through the feeding channel 20 and into the sample-receiving position shown in Figure 1. The user presses the cap 16 against a body part, such as a finger or arm, to allow the lancet assembly to drive the lancet tip into the skin. The lancet assembly subsequently retracts the lancet tip from the skin.

The pressure ring, if present, squeezes the skin to maximize the quantity of blood formed from a puncture. Once the drop of blood is large enough, the user moves the meter so that the sample-receiving area of the strip contacts the blood drop which will be wicked into the strip. The test strip automatically directs the blood sample to an analysis portion and the analysis of the blood sample begins automatically.

After the analysis is complete, the user may open the cap 16 and remove the lancet 13 from the lancet holder. The user may then discard the lancet 13, if desired. Those skilled in the art will recognize that alternate lancet assemblies may be utilized in accordance with the teachings of the present invention. For example, the present invention is not limited to the dual-spring drive train of the illustrative embodiment of the invention.

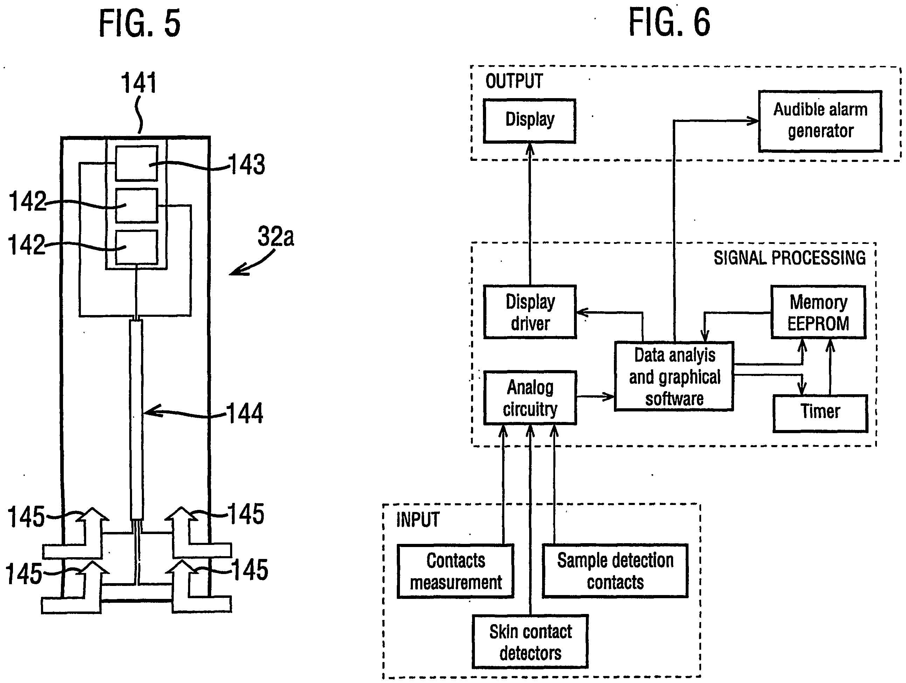

Figure 5 illustrates a test strip design suitable for use in the present invention. The teststrip may utilize OneTouch Ultra (available from LifeScan, Inc. of Milpitas, CA, USA) technology, membrane strip technology or other test strip designs known in the art for electrochemical or photometric analysis of a fluid. According to one embodiment, the test strip includes, as its sample-receiving area, a channel entrance 141 for directing a blood sample to an analysis portion of the strip. The test strip essentially comprises an electrochemical cell, including one or more working electrodes 142 which convert a chemical change, produced by a reaction of glucose or other analyte in the blood sample, to a current. The test strip further includes a reference electrode 143 as a standard to measure the potential of the working electrodes. Leads 144 connect the electrodes to contact bars 145 configured to connect with the electrical contacts of the integrated testing meter. The test strip thus generates a signal indicative of the level of glucose or other analyte in the blood and transmits this signal to the electronics of the device for processing. Those skilled in the art will recognize that a variety of test strip designs and configurations are available in accordance with the teachings of the present invention.

Figure 6 shows a schematic representation of the electronics incorporated in the integrated meter of the present invention. The electronics receive a signal from the electrical contacts, process the signal and transmit instructions for an appropriate display to the display of the device. As shown, input signals related to the electrochemical analysis of the sample are provided from the test strip to a signal processing system. The signals are transmitted via analog circuitry to a processor, which performs data analysis. The processor provides a signal to a display driver connected to an output display. The processor may also provide a signal to an alarm generator. The display and the alarm generator together constitute the output portion of the device. The data analysis processor also communicates with a memory module, such as an EEPROM, in which information, including calibration information and previous test results, may be stored.

According to one practice of the invention, the electronics further include a detector for sensing a strip in the feeding channel. The detector can be two contacts which are shorted by a conductive layer on a strip when the strip is in the sample-receiving position. The electronics may be designed to produce an audible beep or visible signal to indicate to the user that a sufficient sample has been obtained and that analysis is complete. The electronics may also read, store and/or display information regarding the date and time of testing, the condition of the strips, the number of strips remaining in the stack, a calibration code for the strips, the expiration date of the test strip cartridge, the battery power of the meter, and so on. As noted above, test strip specific information can be read directly from the cartridge, for instance by use of bar codes, resistance bridges or memory modules, preferably rewritable memory modules.

As discussed, according to one embodiment, the electronics are switched on when a user slides the knob of the integrated testing meter, or when a test strip detector detects a test strip loaded in the sample-receiving position. Preferably, each time the electronics are switched on, the data on the cartridge are read to ensure that the correct calibration code and other data are used to control the meter. This ensures that a correct test result can be obtained even if the cartridge has been changed.

According to another embodiment of the invention, the electronics are switched off when the user replaces the test strip cartridge cap and ejects the used test strip from the meter. This provides an extra safety feature as it ensures that the cartridge remains closed for as long as possible. This minimises the exposure of the contents of the cartridge to the atmosphere. Preferably, the electronics in the meter are arranged to record the length of time between a strip reaching the sample-receiving position and its being ejected from the meter. This is a measure of the time the cap is open. If the total time the cap is open exceeds a predetermined value, the electronics may be arranged to provide an audible or visible warning signal. The electronics may also be arranged to provide such a signal, or to switch off the meter, if any single strip has remained in the sample-receiving position for longer than a predetermined time.

The integrated meter of the present invention and its components provide significant improvements to the detection and monitoring of glucose levels in the blood. The present invention considerably reduces the pain and inconvenience associated with glucose monitoring. The invention further improves the efficiency and accuracy of testing by providing an automated transfer and analysis of the sample. The invention provides an integrated testing meter with user-friendly, uncomplicated operation. The integrate testing meter is compact, ergonomically sound, discrete and adjustable to different users and body parts while simultaneously providing fast and accurate results.

The present invention achieves a reduction in the pain associated with testing in a number of ways. Shallower punctures of the skin can be used to achieve a sufficient blood sample, reducing painful deep punctures in sensitive body parts. The present invention does not require large sample volumes for analysis. The pressure device, if used, for instance formed by the pressure ring on the cap, provides a high yield from a small puncture. The integrated sapling and testing feature further ensures full usage of the obtained sample and limits "leftovers" on the skin. In current systems, complex and inaccurate sample transfer from a sampling point to a sample-receiving area on a test strip requires surplus sample due to poor utilization of an obtained sample drop. The present invention decreases this inefficiency of transferring samples and provides optimal utilization of the obtained sample by easy direction of the sample to a precise location on the test strip. Optimal utilization of the sample drop reduces the number of attempts needed to provide enough sample for efficient analysis, thus reducing the number of punctures required. The superficial punctures reduce agitation of nerve endings in the skin and reduce pain in sensitive body areas. The variable depth of penetration and the ability to test on a number of different body parts in addition to the finger reduces the concentration and repetition of micro-traumata in a small area, which avoids the problems of tinting, itching, dried and callous skin areas caused by such micro-traumata.

The integrated meter of the present invention is able fully to exploit the technological improvements in strip design which allow the use of much smaller samples. Presently available strips require only 1 to 3 µl of sample. The small volume of blood or other bodily fluid expressed from the user is sufficient to accurately determine or monitor the presence or absence of an analyte, such as glucose.

The present invention further provides easy and uncomplicated operation. The use of the meter significantly reduces the time and difficulty involved in sampling and testing blood. The integrated meter essentially provides three devices, a puncture means, a supply means, a supply of test strips and a meter, within a singular compact housing. Further, the system is designed such that one-handed operation is possible, eliminating the need for a work space or a flat surface. The meter is not subject to human error and inefficiency. Furthermore, the integration of a disposable test strip cartridge makes the loading of a test strip simple, accurate and easy. In current glucose monitoring systems a user requires two hands to load a strip into a glucose meter. However, with the meter of the present invention, the test strip dispensing system automatically loads a test strip in position to receive a blood sample. The present invention also reduced waste by efficiently utilizing available resources. The present invention further protects against compromised test results due to contamination or an improperly calibrated glucose meter.

Typically, the re-writable memory is in the format of an EEPROM (electronically erasable programmable read only memory.) Such a device does not require power to retain the contents of its memory. Examples of re-writable memory modules according to the present invention include EEPROM memory chips such as the AT24C01ASC which is a 1K Bits, (128x8 bytes) two wire serial EEPROM available from Atmel Corporation of San Jose, California USA, in addition to the Fairchild NM93C56M8 which is available from Fairchild Semiconductor Corporation, South Portland, Main, USA and the Xicor X25020P which is available from Xicor, Inc., Milpitas, CA, USA. Examples of Flash EPROM memory chips which might be used in the present invention include the AMD AM29F010-120PC which is available from Advanced Micro Devices, Sunnyvale, California, USA and the Intel P28F020-150V10 which is available from Intel Corporation, Folsom, California, USA.

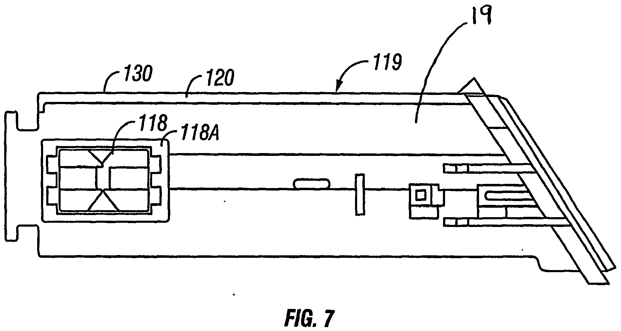

Figure 7, is a further illustration of cartridge 19 wherein re-writable memory module 118 is physically, fixedly connected to a source of test strips, which, in the embodiment of the invention illustrated in Figure 7 is strip cartridge 19, to form an integrated cartridge and memory 119. The source of test strips such as, in this example, strip cartridge 19 may be referred to as a sensor bank. Thus the sensors in strip cartridge 19 are physically associated with re-writable memory module 118 and any information contained within it. Thus, for example, a calibration code written into the re-writable memory module 118 is uniquely associated with the test strips in that bank and meter 10 can read the calibration code from re-writable memory module 118 and use that calibration code in converting a raw test result to a final test result for the test strips stored in cartridge 119. Figure 7 shows in side elevation view a close up of one side of integrated cartridge and memory 119 wherein re-writable memory module 118 located in a matchingly sized recess 118A on the side of housing 120. Re-writable memory module 118 is typically glued by an adhesive to the side of cartridge 19.

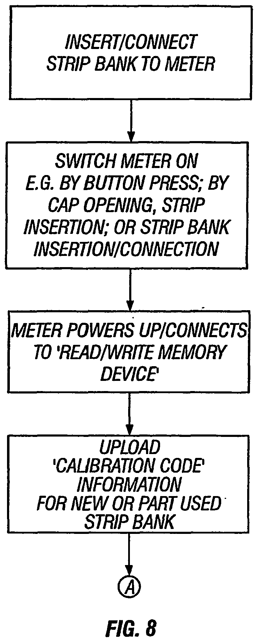

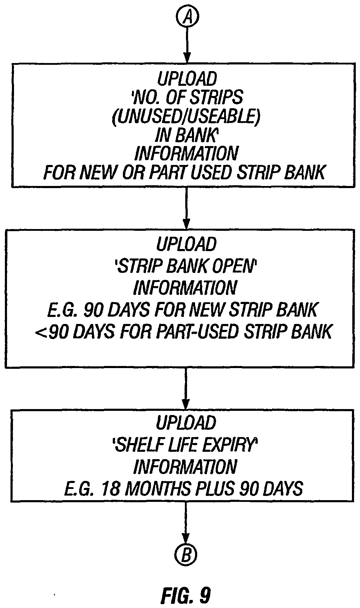

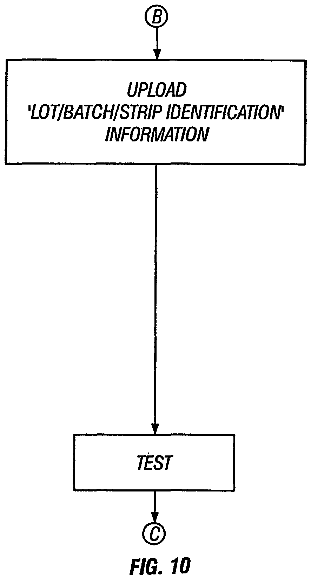

Figures 8, 9 and 10 illustrate a series of steps, any of which may be optional, as will be understood by those more skilled in the art, which might be followed on insertion or connection of an integrate cartridge and memory 119 into a meter 10. It will be understood by those skilled in the art from the information contained herein that one or more items of information is typically uploaded from the strip bank smart chip into the meter. Typically this information will be the calibration code, thus rendering the step of calibrating the meter to a new batch of strips, transparent to a user. If a strip bank is removed or disconnected from the meter when only some of the strips have been used, then a useful application of the present invention is downloading of the number of strips remaining in the strip bank to re-writeable memory module 118. On re-insertion of cassette 19 number of strips remaining can be uploaded from re-writable memory module 118. Other possibilities include the use of a strip bank in which only some of the strips are usable. These strips can be located and used if information concerning the location and type of usable strips is contained within the smart chip. In addition, re-writable memory module 118 may be used to retain information on the date the cassette was first opened and/or shelf life expiration information to the meter in determining whether particular strips should be used or a cassette replaced.

In conclusion, the integrated meter of the present invention significantly reduced the obstacles associated with frequent glucose monitoring. The present invention promotes frequent monitoring for diabetic individuals by providing a simple, efficient, fast and accurate integrated meter.

Since certain changes may be made in the above constructions without departing from the scope of the invention, it is intended that all matter contained in the above description or shown in the accompanying drawings be interpreted as illustrative and not in a limiting sense.

It is also to be understood that the following claims are to cover all generic and specific features of the invention described herein, and all statements of the scope of the invention which, as a matter of language, might be said to fall therebetween.

## Claims

1. An integrated sample-testing meter (10) comprising a single modular housing (11) carrying:
a puncture means (13);
a drive train (14) adapted to drive the puncture means between an extended position and a retracted position;
a test strip cartridge (19) containing a plurality of test strips, each strip having a sample-receiving area;
a sensor configured to analyze a fluid sample received on a test strip;
a test strip dispensing system for moving test strips individually from the cartridge to a sample-receiving position in which the test strip is connected to the sensor;
an assembly adapted to cock the drive train; and
a trigger (21) adapted to activate the cocked drive train and move the puncture means to its extended position to form a puncture in the user's skin, wherein the drive train is adapted to retract the puncture means after it has reached its extended position,
**characterised in that** the assembly is adapted to cock the drive train whilst simultaneously moving a test strip into the sample receiving position for acquiring a sample after the drive train is subsequently activated.

2. The meter of claim 1, which includes electrical or electronic circuitry (22) for controllimg its operation.

3. The meter of claim 2, wherein the circuitry includes a visual display unit (34) from which the user can read out the result of any particular test.

4. The meter of claim 2 or claim 3, wherein the circuitry includes means (35) for entering data into the meter.

5. The meter of any one of claims 1 to 4, which includes a pressure device arranged to facilitate the formation of a drop of fluid around the puncture.

6. The meter of claim 5, wherein the pressure device comprises a pump adapted to apply a negative pressure to a volume in the meter having an aperture for location on the skin of the user.

7. The meter of claim 6, wherein the aperture through which the puncture means extends is made of a non-slip material.

8. The meter of claim 5, wherein the pressure device comprises a pressure ring arranged to be located, in use, on the user's skin and to apply pressure at the edges of the ring to increase the amount of fluid available at the centre of the ring.

9. The meter of claim 8, wherein the pressure ring is made of a non-slip material.

10. The meter of claim 8 or claim 9, wherein the pressure ring is shaped to conform to the shape of the area of the skin to which it is applied,

11. The meter of any one of claims 8 to 10, wherein the pressure ring has a multi-contoured surface to increase the pressure gradient from the outside to the inside of the ring.

12. The meter of any one of claims 8 to 11, wherein the pressure ring is part of a cap which cover the puncture means in its retracted position.

13. The meter of any one of claims 1 to 11, which includes a cap which cover the puncture means in its retracted position.

14. The meter of claim 12 or claim 13, wherein the cap is detachably mounted on the housing.

15. The meter of any one of claims 1 to 14, wherein the drive train is spring driven.

16. The meter of any one of claims 1 to 15, wherein the drive train includes an adjustment screw which allows the user to set the extended position of the puncture means.

17. The meter of claim 16, wherein the operation of the adjustment screw is arranged such that the distance of travel of the puncture means remains constant, however much the extended position of the puncture means is changed.

18. The meter of claim 12 or claim 13 or any claim dependent thereon, wherein the cap provides a means for guiding the drive train so that the puncture means punctures the skin at approximately the same place on each actuation of the drive train.

19. The meter of any one of claims 1 to 18, wherein the test strip cartridge comprises a cartridge housing defining a cavity configured to receive a stack of test strips, a partially detachable cartridge cap and a means for moving the stack strips towards the cartridge cap.

20. The meter of claim 19, wherein the cartridge has on its data relating to the calibration code for the strips in the cartridge.

21. The meter of claim 20, wherein the data on the cartridge are present in machine-readable format.

22. The meter of claim 21, wherein the data are present in an electronic memory module.

23. The meter of any one of claims 20 to 23, wherein the data also include a unique number identifying the specific cartridge, the number of strips originally present in the cartridge, the expiry date for the cartridge, and/or different calibration factors for different sources of fluid.

24. The meter of claim 22 or claim 23 when dependent on claim 22, wherein the memory module on the cartridge is rewritable and the meter is arrange to write back into the memory module information, regarding operation of the meter.

25. The meter of any one of claims 1 to 24, wherein the meter includes a feeding channel which receives the strip from the cartridge and guides it to the sample-receiving position.

26. The meter of any one of claims to 25, which includes an ejection means for ejecting a used test strip from the meter once a test has been completed.

27. The meter of any one of claims 1 to 26, which includes a deviator which prevents the test strip dispensing system moving a further test strip-into the sample-receiving position while a first test strip is still in position.

28. The meter of any one of claim 2 or claims 3 to 27, when dependent on claim 2, which includes a means for verifying that a strip is in the sample-receiving position.

29. The meter of claim 28, wherein the verifying means is also used to activate fully the circuitry in the meter.

30. The meter of any one of claims 1 to 29 which is activated manually by use of a single movement of multi-functional assembly carried by the housing.

31. The meter of any one of claims 1 or 2 or claims 3 to 30, when dependent on claim 2, wherein the strips are adapted to carry out electrochemical analyses and the circuitry in the meter is arranged to contact the electrodes in such strips.

32. The meter of any one of claims 1 to 31, which is adapted to carry out blood glucose testing.

## Patentansprüche

1. Integriertes Probennahme-Messgerät (10) mit einem einzelnen modularen Gehäuse (11), das Folgendes trägt:
ein Punktionsmittel (13);
eine Antriebseinheit (14) zum Antreiben des Punktionsmittels zwischen einer ausgefahrenen Stellung und einer zurückgezogenen Stellung;
eine Teststreifenkartusche (19) mit einer Vielzahl von Teststreifen, wobei jeder Streifen einen Probenaufnahmebereich aufweist;
ein Sensor zur Analyse einer Flüssigkeitsprobe, die auf einem Teststreifen aufgenommen wurde;
ein Teststreifen-Spendersystem zum individuellen Bewegen der Teststreifen von der Kartusche in eine Probenaufnahmestellung, in welcher der Teststreifen mit dem Sensor verbunden ist;
eine Anordnung zum Schieflegen der Antriebseinheit; und
einen Trigger (21) zur Aktivierung der schiefgelegten Antriebseinheit und zur Bewegung des Punktionsmittels in seine ausgefahrene Stellung zur Bildung einer Punktion in der Haut des Anwenders, worin die Antriebseinheit das Punktionsmittel zurückziehen kann, nachdem es seine ausgefahrene Stellung erreicht hat,
**dadurch gekennzeichnet, dass** die Anordnung die Antriebseinheit schieflegen kann, während sie gleichzeitig einen Teststreifen zur Erfassung einer Probe nach anschließender Aktivierung der Antriebseinheit in die Probenaufnahmestellung bewegt.

2. Messgerät nach Anspruch 1, das einen elektrischen oder elektronischen Kreislauf (22) zur Steuerung seines Betriebs aufweist.

3. Messgerät nach Anspruch 2, worin der Kreislauf eine visuelle Anzeigeeinheit (34) aufweist, von welcher der Anwender das Ergebnis eines bestimmten Tests ablesen kann.

4. Messgerät nach Anspruch 2 oder 3, worin der Kreislauf ein Mittel (35) zur Eingabe von Daten in das Messgerät aufweist.

5. Messgerät nach einem der Ansprüche 1 bis 4, das eine Druckvorrichtung zur Erleichterung der Bildung eines Flüssigkeitstropfens um die Einstichstelle aufweist.

6. Messgerät nach Anspruch 5, worin die Druckvorrichtung eine Pumpe umfasst, die einen Unterdruck auf ein Volumen im Messgerät aufbringen kann und eine Öffnung zur Anordnung auf der Haut des Anwenders aufweist.

7. Messgerät nach Anspruch 6, worin die Öffnung, durch die sich das Punktionsmittel erstreckt, aus einem rutschfesten Material besteht.

8. Messgerät nach Anspruch 5, worin die Druckvorrichtung einen Druckring umfasst, der im Gebrauch auf der Haut des Anwenders angeordnet wird und Druck an den Rändern des Rings aufbringt, um die in der Mitte des Rings vorhandene Flüssigkeitsmenge zu erhöhen.

9. Messgerät nach Anspruch 8, worin der Druckring aus einem rutschfesten Material besteht.

10. Messgerät nach Anspruch 8 oder 9, worin der Druckring so geformt ist, dass er sich an die Form des Hautbereichs, an den er angelegt wird, anpasst.

11. Messgerät nach einem der Ansprüche 8 bis 10, worin der Druckring eine Oberfläche mit mehreren Konturen zur Erhöhung des Druckgefälles von der Außenseite zur Innenseite des Rings aufweist.

12. Messgerät nach einem der Ansprüche 8 bis 11, worin der Druckring Teil einer Kappe ist, die das Punktionsmittel in seiner zurückgezogenen Stellung abdeckt.

13. Messgerät nach einem der Ansprüche 1 bis 11, das eine Kappe aufweist, die das Punktionsmittel in seiner zurückgezogenen Stellung abdeckt.

14. Messgerät nach Anspruch 12 oder 13, worin die Kappe lösbar auf dem Gehäuse angeordnet ist.

15. Messgerät nach einem der Ansprüche 1 bis 14, worin die Antriebseinheit von einer Feder angetrieben wird.

16. Messgerät nach einem der Ansprüche 1 bis 15, worin die Antriebseinheit eine Stellschraube aufweist, mit welcher der Anwender die ausgefahrene Stellung des Punktionsmittels fixieren kann.

17. Messgerät nach Anspruch 16, worin die Stellschraube so bedient wird, dass die Bewegungsstrecke des Punktionsmittels konstant bleibt, aber die ausgefahrene Stellung des Punktionsmittels verändert wird.

18. Messgerät nach Anspruch 12 oder 13 oder einem davon abhängigen Anspruch, worin die Kappe ein Mittel zur Führung der Antriebseinheit bietet, so dass das Punktionsmittel die Haut bei jeder Betätigung der Antriebseinheit ungefähr an derselben Stelle einsticht.

19. Messgerät nach einem der Ansprüche 1 bis 18, worin die Teststreifenkartusche ein Kartuschengehäuse, das einen Hohlraum zur Aufnahme eines Teststreifenstapels definiert, eine teilweise abnehmbare Kartuschenkappe und ein Mittel zum Bewegen des Streifenstapels zur Kartuschenkappe umfasst.

20. Messgerät nach Anspruch 19, worin die Kartusche Daten in Bezug auf den Kalibrierungscode für die Streifen in der Kartusche trägt.

21. Messgerät nach Anspruch 20, worin die Daten auf der Kartusche in einem maschinenlesbaren Format vorliegen.

22. Messgerät nach Anspruch 21, worin die Daten in einem elektronischen Speichermodul vorliegen.

23. Messgerät nach einem der Ansprüche 20 bis 23, worin die Daten auch eine eindeutige Nummer zur Identifizierung der jeweiligen Kartusche, der ursprünglich in der Kartusche vorliegenden Streifenanzahl, des Verfalldatums der Kartusche und/oder von verschiedenen Kalibrationsfaktoren für unterschiedliche Flüssigkeitsquellen aufweist.

24. Messgerät nach Anspruch 22 oder 23, wenn von Anspruch 22 abhängig, worin das Speichermodul auf der Kartusche überschreibbar ist und das Messgerät Informationen in Bezug auf die Bedienung des Messgeräts in das Speichermodul schreiben kann.

25. Messgerät nach einem der Ansprüche 1 bis 24, worin das Messgerät einen Zuführungskanal aufweist, der den Streifen von der Kartusche empfängt und ihn in die Probenaufnahmestellung führt.

26. Messgerät nach einem der Ansprüche 1 bis 25, das ein Auswurfmittel zum Auswerfen eines gebrauchten Teststreifens aus dem Messgerät nach Abschluss des Tests aufweist.

27. Messgerät nach einem der Ansprüche 1 bis 26, das einen Deviator aufweist, der verhindert, dass das Teststreifen-Spendersystem einen weiteren Teststreifen in die Probenaufnahmestellung bewegt, während sich noch ein Teststreifen darin befindet.

28. Messgerät nach einem der Ansprüche 2 oder der Ansprüche 3 bis 27, wenn abhängig von Anspruch 2, das ein Mittel zur Verifizierung, dass sich ein Teststreifen in der Probenaufnahmestellung befindet, aufweist.

29. Messgerät nach Anspruch 28, worin das Verifizierungsmittel auch zur völligen Aktivierung des Kreislaufs im Messgerät verwendet wird.

30. Messgerät nach einem der Ansprüche 1 bis 29, das durch Verwendung einer einzigen Bewegung einer multifunktionalen Anordnung, die vom Gehäuse getragen wird, manuell aktiviert wird.

31. Messgerät nach einem der Ansprüche 1 oder 2 oder der Ansprüche 3 bis 30, wenn abhängig von Anspruch 2, worin die Streifen elektrochemische Analysen durchführen können und der Kreislauf im Messgerät mit den Elektroden in solchen Streifen in Kontakt kommt.

32. Messgerät nach einem der Ansprüche 1 bis 31, das Blutzuckertests durchführen kann.

## Revendications

1. Appareil intégré d'analyse d'échantillons (10) comportant un seul logement (11) modulaire portant :
un moyen (13) de perforation ;
un dispositif (14) d'entraînement conçu pour entraîner le moyen de perforation entre une position déployée et une position rétractée ;
une cartouche (19) de bandelettes réactives contenant une pluralité de bandelettes réactives, chaque bandelette étant munie d'une zone de réception d'échantillon ;
un capteur configuré pour analyser un échantillon de fluide reçu sur une bandelette réactive ;
un système de distribution de bandelettes réactives pour déplacer individuellement les bandelettes réactives de la cartouche à une position de réception d'échantillon dans laquelle la bandelette réactive est reliée au capteur ;
un assemblage conçu pour armer le dispositif d'entraînement ; et
un déclencheur (21) conçu pour activer le dispositif d'entraînement armé et déplacer le moyen de perforation à sa position déployée de façon à effectuer une perforation sur la peau de l'utilisateur, le dispositif d'entraînement étant conçu pour rétracter le moyen de perforation une fois la position déployée de ce dernier atteinte,
**caractérisé en ce que** l'assemblage est conçu pour armer le dispositif d'entraînement tout en déplaçant simultanément une bandelette réactive dans la position de réception d'échantillon afin d'acquérir un échantillon après l'activation subséquente du dispositif d'entraînement.

2. Appareil d'analyse selon la revendication 1, qui comprend des circuits (22) électriques ou électroniques pour commander son fonctionnement.

3. Appareil d'analyse selon la revendication 2, dans lequel les circuits comprennent un écran (34) de visualisation sur lequel l'utilisateur peut lire les résultats de n'importe quelle analyse particulière.

4. Appareil d'analyse selon la revendication 2 ou la revendication 3, dans lequel les circuits comprennent un moyen (35) pour saisir des données dans l'appareil d'analyse.

5. Appareil d'analyse selon l'une quelconque des revendications 1 à 4, qui comprend un dispositif de pression agencé pour faciliter la formation d'une goutte de fluide autour de la perforation.

6. Appareil d'analyse selon la revendication 5, dans lequel le dispositif de pression comporte une pompe conçue pour appliquer une pression négative sur un volume dans l'appareil d'analyse pourvu d'une ouverture pour la placer sur la peau de l'utilisateur.

7. Appareil d'analyse selon la revendication 6, dans lequel l'ouverture à travers laquelle s'étend le moyen de perforation est fabriquée dans un matériau non glissant.

8. Appareil d'analyse selon la revendication 5, dans lequel le dispositif de pression comporte une bague de pression agencée pour être placée, pendant l'utilisation, sur la peau de l'utilisateur et pour appliquer une pression au niveau des bords de la bague afin d'augmenter la quantité de liquide disponible au niveau du centre de la bague.

9. Appareil d'analyse selon la revendication 8, dans lequel la bague de pression est fabriquée dans un matériau non glissant.

10. Appareil d'analyse selon la revendication 8 ou la revendication 9, dans lequel la bague de pression est façonnée de façon à se conformer à la forme de la zone de la peau sur laquelle elle est appliquée.

11. Appareil d'analyse selon l'une quelconque des revendications 8 à 10, dans lequel la bague de pression présente une surface à contours multiples pour accroître le degré de pression de l'extérieur vers l'intérieur de la bague.

12. Appareil d'analyse selon l'une quelconque des revendications 8 à 11, dans lequel la bague de pression fait partie d'un capuchon qui recouvre le moyen de perforation dans sa position rétractée.

13. Appareil d'analyse selon l'une quelconque des revendications 1 à 11, qui comprend un capuchon recouvrant le moyen de perforation dans sa position rétractée.

14. Appareil d'analyse selon la revendication 12 ou la revendication 13, dans lequel le capuchon est monté de manière amovible sur le logement.

15. Appareil d'analyse selon l'une quelconque des revendications 1 à 14, dans lequel le dispositif d'entraînement est entraîné par ressort.

16. Appareil d'analyse selon l'une quelconque des revendications 1 à 15, dans lequel le dispositif d'entraînement comprend une vis de réglage qui permet à l'utilisateur de définir la position déployée du moyen de perforation.

17. Appareil d'analyse selon la revendication 16, dans lequel le fonctionnement de la vis de réglage est agencé de sorte que la distance de déplacement du moyen de perforation reste constante, quelle que soit l'ampleur du changement de la position déployée du moyen de perforation.

18. Appareil d'analyse selon la revendication 12 ou la revendication 13 ou l'une quelconque des revendications selon laquelle elle dépend, dans lequel le capuchon fournit un moyen de guidage du dispositif d'entraînement de façon à ce que le moyen de perforation perfore la peau à peu près au même endroit à chaque actionnement du dispositif d'entraînement.

19. Appareil d'analyse selon l'une quelconque des revendications 1 à 18, dans lequel la cartouche de bandelettes réactives comporte un logement de cartouche délimitant une cavité configurée pour recevoir une pile de bandelettes réactives, un capuchon de cartouche partiellement amovible et un moyen de déplacement de la pile de bandelettes vers le capuchon de cartouche.

20. Appareil d'analyse selon la revendication 19, dans lequel la cartouche a sur elle ses données se rapportant au code d'étalonnage pour les bandelettes dans la cartouche.

21. Appareil d'analyse selon la revendication 20, dans lequel les données sur la cartouche sont présentes dans un format lisible par machine.

22. Appareil d'analyse selon la revendication 21, dans lequel les données sont présentes dans un module de mémoire électronique.

23. Appareil d'analyse selon l'une quelconque des revendications 20 à 23, dans lequel les données comprennent aussi un numéro unique identifiant la cartouche spécifique, le nombre de bandelettes présentes initialement dans la cartouche, la date d'échéance de la cartouche, et/ou différents facteurs d'étalonnage pour différentes sources de fluides.

24. Appareil d'analyse selon la revendication 22 ou la revendication 23 lorsque dépendant de la revendication 22, dans lequel le module de mémoire sur la cartouche est réinscriptible et l'appareil d'analyse est agencé pour réécrire dans le module de mémoire les informations relatives au fonctionnement de l'appareil d'analyse.

25. Appareil d'analyse selon l'une quelconque des revendications 1 à 24, dans lequel l'appareil d'analyse comprend un canal d'alimentation qui reçoit la bandelette depuis la cartouche et la guide vers la position de réception d'échantillon.

26. Appareil d'analyse selon l'une quelconque des revendications 1 à 25, qui comprend un moyen d'éjection d'une bandelette réactive usagée d'un appareil d'analyse une fois l'analyse terminée.

27. Appareil d'analyse selon l'une quelconque des revendications 1 à 26, qui comprend un dispositif de déviation qui empêche le système de distribution de bandelettes réactives de déplacer une autre bandelette réactive dans la position de réception d'échantillon lorsqu'une première bandelette réactive est encore position.

28. Appareil d'analyse selon la revendication 2 ou l'une quelconque des revendications 3 à 27, lorsque dépendante de la revendication 2, qui comprend un moyen de vérification qu'une bandelette est dans la position de réception d'échantillon.

29. Appareil d'analyse selon la revendication 28, dans lequel le moyen de vérification est aussi utilisé pour activer entièrement les circuits dans l'appareil d'analyse.

30. Appareil d'analyse selon l'une quelconque des revendications 1 à 29 qui est activé manuellement par l'utilisation d'un seul déplacement de l'assemblage multifonctionnel porté par le logement.

31. Appareil d'analyse selon l'une quelconque des revendications 1 ou 2 ou des revendications 3 à 30, lorsque dépendante de la revendication 2, dans lequel les bandelettes sont conçues pour réaliser des analyses électrochimiques et les circuits dans l'appareil d'analyse sont agencés pour établir un contact avec les électrodes dans ces bandelettes.

32. Appareil d'analyse selon l'une quelconque des revendications 1 à 31, qui est conçu pour réaliser des analyses du glucose dans le sang.
